# EUROPEAN PATENT APPLICATION

(11) **EP 1 666 068 A1**
(43) Date of publication of application: **07.06.2006**
(21) Application number: 04773534.5
(22) Date of filing: 24.09.2004
(51) Int. Cl.: A61K 45/00, A61K 38/05, A61K 38/06, A61K 38/08, A61P 9/10, A61P 25/02, A61P 27/02, A61P 27/06, A61P 43/00

(54) **REMEDY FOR EYE DISEASES ACCOMPANIED BY OPTIC NERVE INJURIES**

(30) Priority: 24.09.2003 JP 2003331122; 23.04.2004 JP 2004128218
(71) Applicant: SANTEN PHARMACEUTICAL CO., LTD., Higashiyodogawa-ku, Osaka-shi, Osaka 533-8651 (JP)
(72) Inventor: YONEDA, Shinji, SANTEN PHARMACEUTICAL CO., LTD., Ikoma-shi, Nara 6300101 (JP); YAMAMOTO, Rie, SANTEN PHARMACEUTICAL CO., LTD., Ikoma-shi, Nara 6300101 (JP); HARA, Hideaki, SANTEN PHARMACEUTICAL CO., LTD., Ikoma-shi, Nara 6300101 (JP)
(74) Representative: Peaucelle, Chantal
(86) International application number: PCT/JP2004/014446
(87) International publication number: WO 2005/027969

(57) **Abstract**

An object of the present invention is to search for a novel medicinal use of a compound having a secretase inhibitory activity. Because the compound having a secretase inhibitory activity has a remarkable inhibitory effect on retinal neuronal cell death, it is useful as a therapeutic agent for a retinal disease typified by retinal vessel occlusion, diabetic retinopathy, ischemic optic neuropathy, macular degeneration, retinitis pigmentosa and Leber's disease or an eye disease accompanied by an optic neuropathy such as glaucoma.

## Description

### Technical Field

The present invention relates to a protective agent for retinal neurons comprising a secretase inhibitor as an active ingredient and in particular to a therapeutic agent for an eye disease accompanied by an optic neuropathy such as a retinal disease or glaucoma.

### Background Art

The retina has the function of receiving light from outside and plays an important role for visual function. Structurally, it is a tissue with a thickness of 0.1 mm to 0.5 mm consisting of 10 layers including a pigmented epithelial layer of retina, an inner plexiform layer, a retinal ganglion cell layer, a retinal nerve fiber layer and others. The inner plexiform layer has neurons, which are called an amacrine cell and form a synapse by forming a pair with a ganglion cell projection. Because the neurons are highly responsive at the time of the initiation and termination of light illumination, they are thought to function as a detector of light intensity. The retinal ganglion cell layer has retinal ganglion cells (hereinafter referred to as "RGCs"), which are located in the most inner side of the retina. These cells are deeply involved in motion vision, peripheral vision, color vision, perception of form and the like. Further, in the retinal nerve fiber layer, a retinal vessel, which is a branch of the central retinal artery and vein, is running and has the role of supplying oxygen and nutrition to retinal neurons.

When the retinal vessel is occluded or constricted due to causes including spasms, thrombi, arterioscleroses and the like, a disorder in retinal circulation occurs and the supply of oxygen and nutrition to a retinal neuron is cut off. The retinal circulatory disorder is particularly important among retinal diseases, and if oxygen or nutrition is in short supply due to a retinal circulatory disorder, retinal neurons are led to death and optic neuropathy is caused in the end. Typical examples of the symptoms accompanied by the optic neuropathy include retinal vessel occlusion in which a retinal vein or a retinal artery is occluded or constricted, diabetic retinopathy which can cause retinal detachment and ischemic optic neuropathy in which disturbance of visual function appears. Further, the retinal neuronal cell death is thought to be deeply involved in the onset of macular degeneration, retinitis pigmentosa, Leber's disease or the like.

Glaucoma is one of the eye diseases which cause serious disturbance of visual function leading to the blindness if it is not treated appropriately. In glaucoma, RGCs among retinal neurons are selectively injured to cause an optic neuropathy, which progresses to visual field defect. That is because the idea of so-called "neuroprotection" that a treatment for preventing or minimizing RGC injury leads to an ultimate glaucoma treatment is being established (Ophthalmology, 40, 251-273, 1998).

The detailed mechanism of glaucomatous optic neuropathy has not been known yet, but a mechanical disorder theory, in which optic nerve atrophy is caused by the direct compression of the optic nerve due to the increase in the intraocular pressure, and a circulatory disorder theory, in which the main cause of optic nerve atrophy is a circulatory disorder in the optic papilla, have been proposed and it is considered that both of these mechanisms based on a mechanical disorder and a circulatory disorder are related in a complex manner. Further, both the mechanical disorder and the circulatory disorder cause an optic nerve axonal transport disorder, and it is considered that the disruption of supply of a neurotrophic factor accompanying this axonal transport disorder is one of the causes of RGC injury (Ophthalmology, 44, 1413-1416, 2002). In addition, glutamic acid is one of the neurotransmitters in the retina and it is considered that the excess activation of the glutamate signaling cascade by any cause is one of the causes of RGC injury (Surv. Ophthalmol. 48, S38-S46, 2003).

Accordingly, if there is an active compound that has a protective effect on retinal neurons such as RGCs, it is expected to be useful for the treatment of a retinal disease typified by retinal vessel occlusion, diabetic retinopathy, ischemic optic neuropathy, macular degeneration, retinitis pigmentosa and Leber's disease, or an eye disease accompanied by an optic neuropathy such as glaucoma.

On the other hand, α-, β- and γ- subtypes are known as a secretase, and a compound having an inhibitory activity against β- or γ-secretase among them was reported to be effective in the treatment of Alzheimer's disease by suppressing the production of β-amyloid peptide (JP-T-2001-503782 and JP-T-11-506923 (the term "JP-T" as used herein means a "published Japanese translation of a PCT patent application")). However, it has not been studied at all that what pharmacological action a compound having a secretase inhibitory activity exhibits on a retinal disease or an eye disease such as glaucoma.

### Disclosure of the invention

It is a very interesting subject to search for a novel medicinal use of a compound having a secretase inhibitory activity.

The present inventors have performed pharmacological tests for a secretase inhibitor such as a β-secretase inhibitor or a γ-secretase inhibitor using retinal neurons in primary culture, RGCs and rats and found that the secretase inhibitor exhibits an excellent inhibitory effect on cell death, and thus the present invention has been completed.

The present invention is directed to a protective agent for retinal neurons such as RGCs and this protective agent comprises a secretase inhibitor as an active ingredient, wherein the secretase inhibitor is useful as a therapeutic agent for a retinal disease typified by retinal vessel occlusion, diabetic retinopathy, ischemic optic neuropathy, macular degeneration, retinitis pigmentosa and Leber's disease or an eye disease accompanied by an optic neuropathy such as glaucoma.

In the present invention, the secretase inhibitor is not particularly limited as long as it is a compound having a secretase inhibitory activity, and it can be, for example, a β-secretase inhibitor or a γ-secretase inhibitor. Such a secretase inhibitor is described in US 2003/0125257, JP-T-2001-503782, Biochem. Biophys. Res. Commun., 268, 133-135 (2000), Int. J. Pept. Protein Res., 20, 16-25 (1982), JP-A-2002-37731, US 2002/0013315, US 2002/0115616, US 2003/0055005, US 2003/0109559, US 2003/0135044, etc. Specific examples thereof include β-secretase inhibitors such as glutamyl-valyl-asparagyl-3-hydroxy-6-methyl-γ- aminoheptanoyl-valyl-alanyl-glutamyl-phenylalanine amide and N-benzyloxycarbonyl-valyl- leucyl-leucinal, γ-secretase inhibitors such as N-(3,5-difluorophenylacetyl)-L-alanyl-(S)-phenylglycine- methylester and N-benzyloxycarbonyl-S-(t-butyl)cysteyl- isoleucyl-leucinal and the like.

The protective effect of the secretase inhibitor of the present invention on retinal neurons is based on the fact that the secretase inhibitor such as a β-secretase inhibitor or a γ-secretase inhibitor exhibits an excellent inhibitory effect on retinal neuronal cell death in a pharmacological test using cultured rat embryonic retinal neurons in which the cell death has been induced by serum removal, a pharmacological test using cultured rat RGCs in which the cell death has been induced by glutamic acid and a pharmacological test for a retinal disorder induced by the compression of the axon in a rat, which will be described later.

The secretase inhibitor of the present invention can be made into a preparation, a single preparation or a mixed preparation, by adding a pharmaceutically acceptable additive as needed according to a technique which is widely used.

The secretase inhibitor of the present invention can be administered either parenterally or orally. Examples of the dosage form for parenteral administration include eye drops, injections, nasal drops and the like, and examples of the dosage form for oral administration include tablets, capsules, fine granules, granules, powders and the like. These can be prepared by a technique which is widely used. For example, an eye drop can be prepared by adding appropriately, as an additive, a tonicity agent, a buffer, a pH adjusting agent, a solubilizer, a viscous agent, a stabilizer, a preservative or the like. Further, a stable eye drop can also be obtained by adding a pH adjusting agent, a viscous agent, a dispersant or the like to suspend an active ingredient.

Examples of the tonicity agent include glycerin, propylene glycol, sodium chloride, potassium chloride, sorbitol, mannitol and the like.

Examples of the buffer include phosphoric acid, phosphate, citric acid, acetic acid, ε-aminocaproic acid, and the like.

Examples of the pH adjusting agent include hydrochloric acid, citric acid, phosphoric acid, acetic acid, sodium hydroxide, potassium hydroxide, boric acid, borax, sodium carbonate, sodium hydrogencarbonate and the like.

Examples of the solubilizer include polysorbate 80, polyoxyethylene hydrogenated castor oil 60, macrogol 4000 and the like.

Examples of the viscous agent and the dispersant include cellulosic polymers such as hydroxypropylmethyl cellulose and hydroxypropyl cellulose, polyvinyl alcohol, polyvinylpyrrolidone and the like. Examples of the stabilizer include edetic acid, sodium edetate and the like.

Examples of the preservative (antiseptic) include sorbic acid, potassium sorbate, benzalkonium chloride, benzethonium chloride, methyl p-oxybenzoate, propyl p-oxybenzoate, chlorobutanol and the like which are widely used. These preservatives can also be used in combination.

The pH of the eye drop can be within the range which is acceptable for an ophthalmic preparation, however, it is preferably in the range from 4.0 to 8.5. In addition, the osmotic pressure ratio is preferably adjusted at around 1.0. The injection includes solutions, suspensions, emulsions and solid injections which are dissolved or suspended in a liquid when it is used. The injection is prepared, for example, by dissolving, suspending or emulsifying an active ingredient in a liquid.

The dose can properly be selected depending on the symptoms, the age of patients, the dosage form and the like. An eye drop can be instilled once to several times a day at a concentration of from 0.001 to 10% (w/v). In the case of an injection, the secretase inhibitor can be administered once to several times a day in a dose of generally from 0.0001 to 1 mg per day. In the case of an oral preparation, the secretase inhibitor can be administered once to several times a day in a dose of generally from 10 µg to 1 g per day.

As will be described in detail in the section of pharmacological tests below, when the secretase inhibitor was added to a culture medium, cultured retinal neuronal cell death and cultured RGC death could be remarkably inhibited. In addition, when an effect on a retinal disorder induced by the compression of the axon in a rat was examined, the secretase inhibitor exhibited a remarkable inhibitory effect on cell death of RGCs. These demonstrated that the secretase inhibitor of the present invention is useful as a protective agent for a retinal neuron and as a therapeutic agent for a retinal disease typified by retinal vessel occlusion, diabetic retinopathy, ischemic optic neuropathy, macular degeneration, retinitis pigmentosa and Leber's disease or an eye disease accompanied by an optic neuropathy such as glaucoma.

### Brief description of the Drawings

Fig. 1 is a graph showing the survival rate of each group in the case of using GL-189 by serum removal.
Fig. 2 is a graph showing the survival rate of each group in the case of using Z-VLL-CHO by serum removal.
Fig. 3 is a graph showing the survival rate of each group in the case of using DAPM by serum removal.
Fig. 4 is a graph showing the survival rate of each group in the case of using γXIV by serum removal.
Fig. 5 is a graph showing the survival rate of each group in the case of using GL-189 by the addition of glutamic acid.
Fig. 6 is a graph showing the survival rate of each group in the case of using Z-VLL-CHO by the addition of glutamic acid.
Fig. 7 is a graph showing the survival rate of each group in the case of using DAPM by the addition of glutamic acid.
Fig. 8 is a graph showing the survival rate of each group in the case of using γXIV by the addition of glutamic acid.
Fig. 9 is a graph showing the effects of DAPM and Z-VLL-CHO on a retinal disorder induced by the compression of the axon in a rat.

### Best Mode for Carrying Out the Invention

Hereinafter, the present invention will be described in detail with reference to pharmacological tests and preparation examples. However, these examples are for understanding well the invention, and do not limit the scope of the invention.

### [Pharmacological tests]

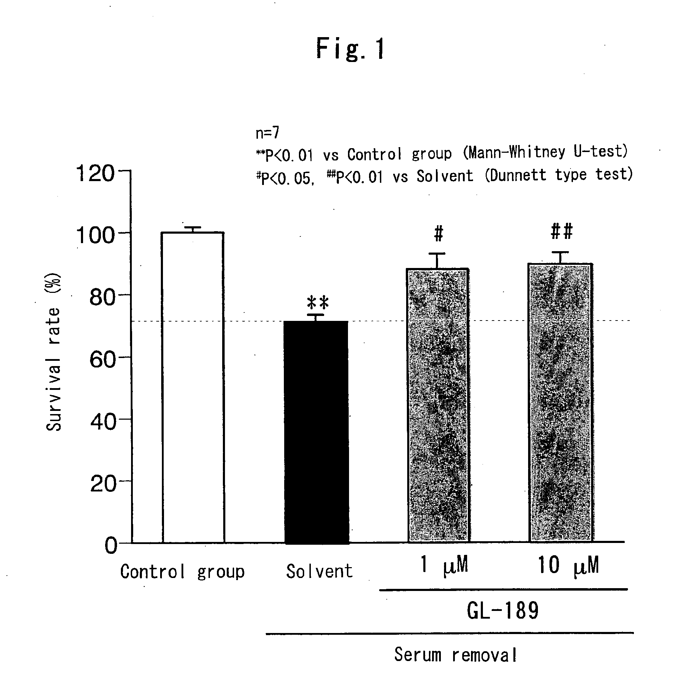

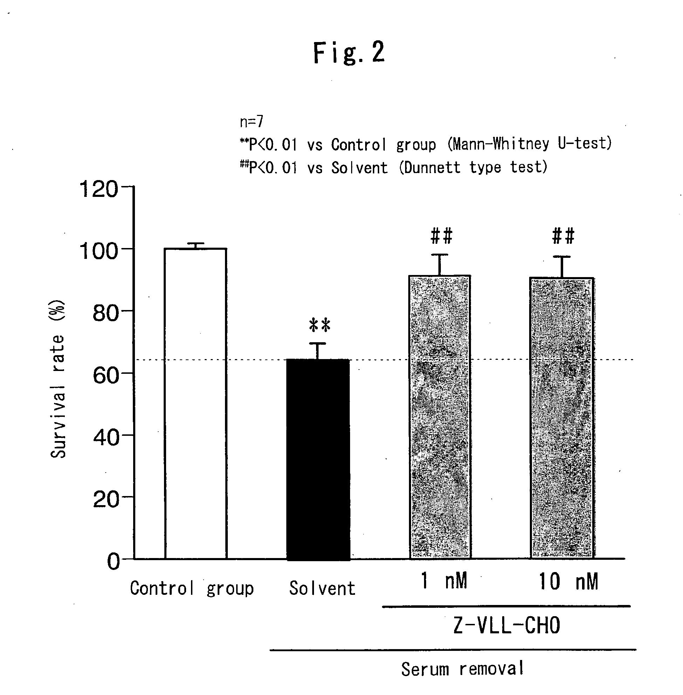

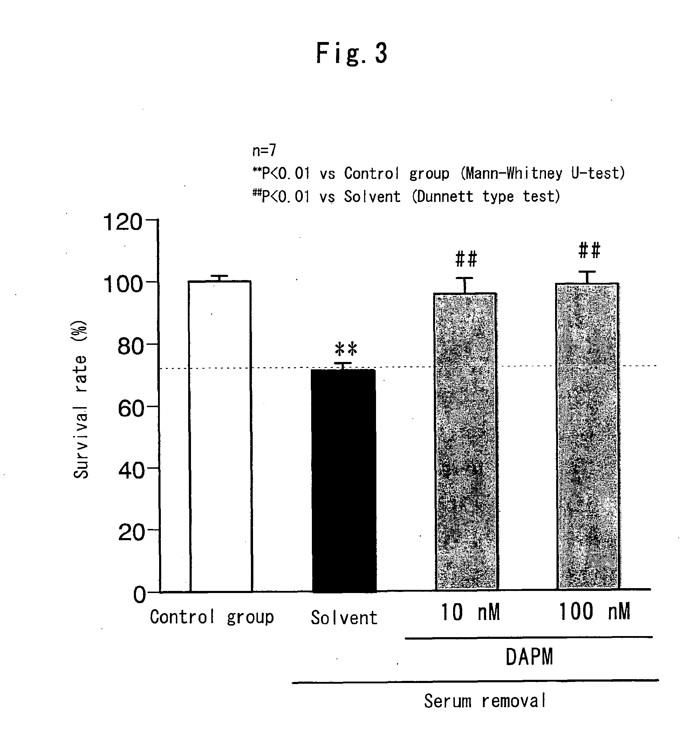

In order to examine the effect of a compound having a secretase inhibitory activity on protecting retinal neurons, by using glutamyl-valyl-asparagyl-3- hydroxy-6-methyl-γ-aminoheptanoyl-valyl-alanyl- glutamyl-phenylalanine amide (hereinafter referred to as "GL-189", manufactured by CALBIOCHEM), which is a β-secretase inhibitor, N-benzyloxycarbonyl-valyl- leucyl-leucinal (hereinafter referred to as "Z-VLL-CHO", manufactured by CALBIOCHEM), which is a β-secretase inhibitor, N-(3,5-difluorophenylacetyl)-L-alanyl-(S)- phenylglycine-methylester (hereinafter referred to as "DAPM", manufactured by CALBIOCHEM), which is a γ-secretase inhibitor and N-benzyloxycarbonyl-S-(t- butyl)cysteyl-isoleucyl-leucinal (hereinafter referred to as "γXIV", manufactured by CALBIOCHEM), which is a γ-secretase inhibitor, (A) an effect on cultured rat embryonic retinal neuronal cell death induced by serum removal, (B) an effect on cultured rat neonatal RGC death induced by the addition of glutamic acid and (C) an effect on a retinal disorder induced by the compression of the axon in a rat were examined by the following methods.
(A) Effect on cultured rat embryonic retinal neuronal cell death induced by serum removal
   (1) Effect of GL-189
      According to the test method described in Brain Res., 967, 257-66, 2003, retinal neurons of a rat embryo (at 18 days of age) were isolated and seeded on a plastic cover slip coated with polyethylenimine. Then, the cells were cultured for 5 days in an Eagle's minimum essential medium containing 10% fetal bovine serum. From day 6 of the culture, the cells were cultured in an Eagle's minimum essential medium containing 10% fetal bovine serum supplemented with a cytosine arabinoside (at a concentration of 10 µM in the medium), whereby the growth of non-neuronal cells was inhibited. On day 8 of the culture, the medium was changed to an Eagle's minimum essential medium containing 10% horse serum and cultivation was carried out for 24 hours, and then the cells were used for the test. Incidentally, the cultivation was carried out under the conditions of 37°C and 5% CO₂. On day 9 of the culture, the retinal neurons were incubated (at 37°C and 5% CO₂) for 24 hours in an Eagle's minimum essential medium which contains a solvent (0.1% dimethylsulfoxide) or GL-189 (1 µM and 10 µM) and does not contain serum. Then, the cell viability was determined, and the survival rate of each group to that of the control group (100%) incubated for 24 hours in an Eagle's minimum essential medium containing 10% horse serum supplemented with 0.1% dimethylsulfoxide was calculated. The cell viability was determined by the trypan blue staining method. More specifically, the cells were stained for 10 minutes with 1.5% trypan blue solution, fixed with 10% neutral formalin solution and washed with a physiological saline. A stained cell was determined as a dead cell and an unstained cell was determined as a live cell, and the numbers of dead cells and live cells (the total number of cells was 200 or more) were counted under a microscope. The experimental results are shown in the graph of Fig. 1. The columns in the graph represent the average value for each group, and the error lines represent the standard error.
   (2) Effect of Z-VLL-CHO
      The same procedure as that described in (1) above was followed except that Z-VLL-CHO (1 nM and 10 nM) was used instead of GL-189, and the survival rate of each group was calculated. The experimental results are shown in the graph of Fig. 2.
   (3) Effect of DAPM
      The same procedure as that described in (1) above was followed except that DAPM (10 nM and 100 nM) was used instead of GL-189, and the survival rate of each group was calculated. The experimental results are shown in the graph of Fig. 3.
   (4) Effect of γXIV

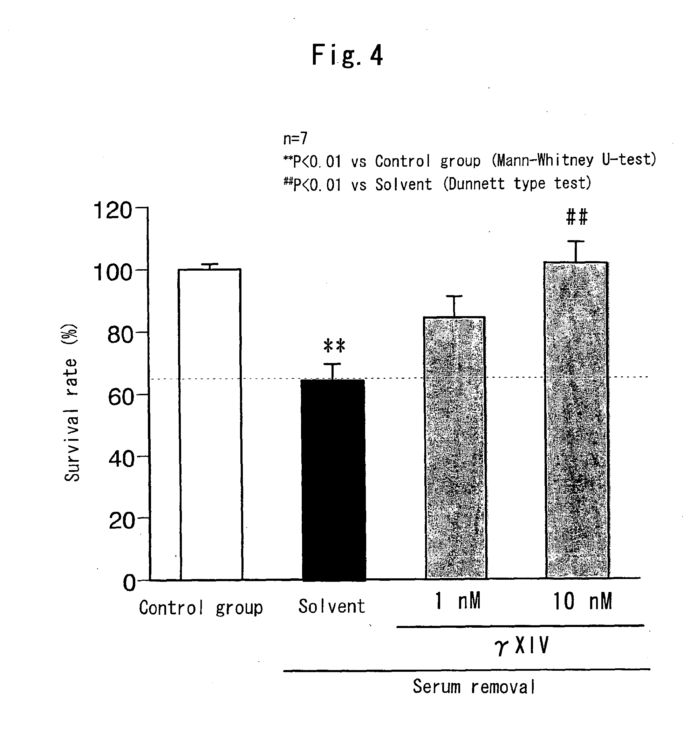

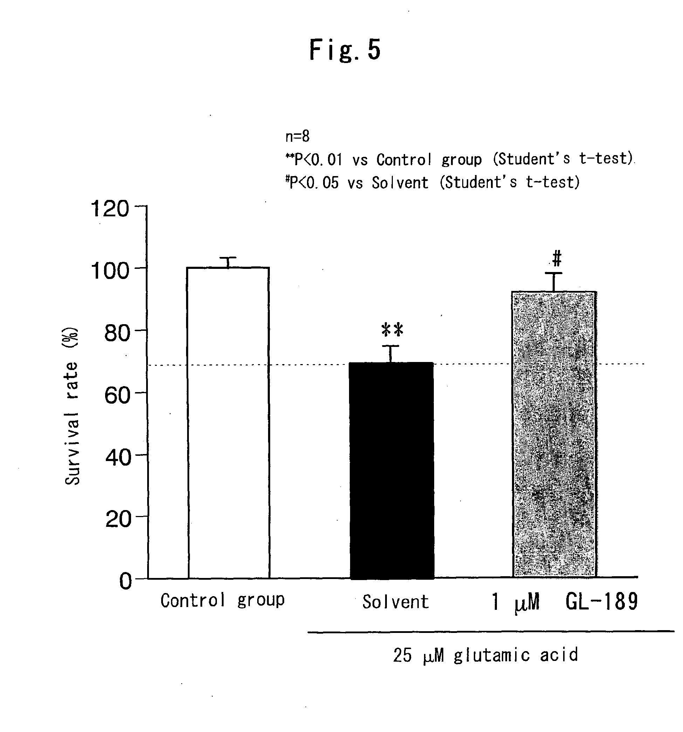

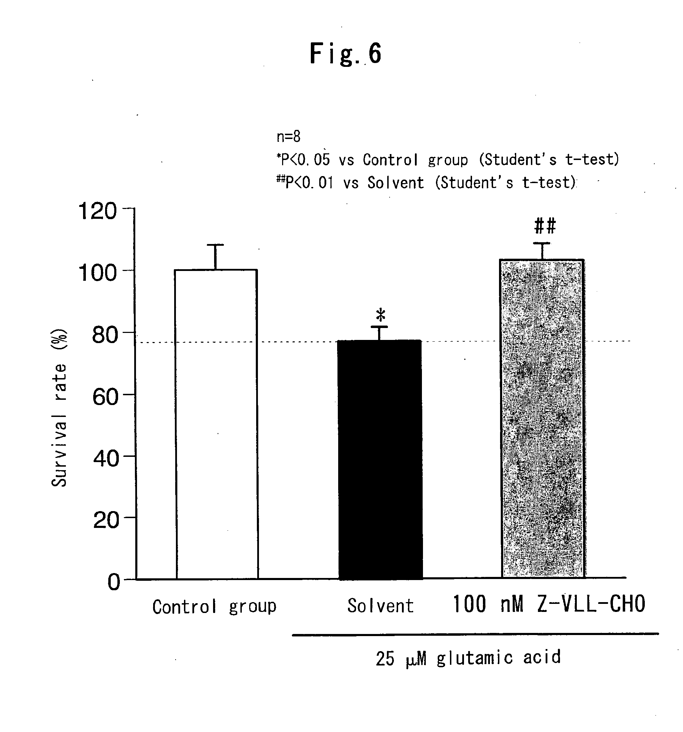

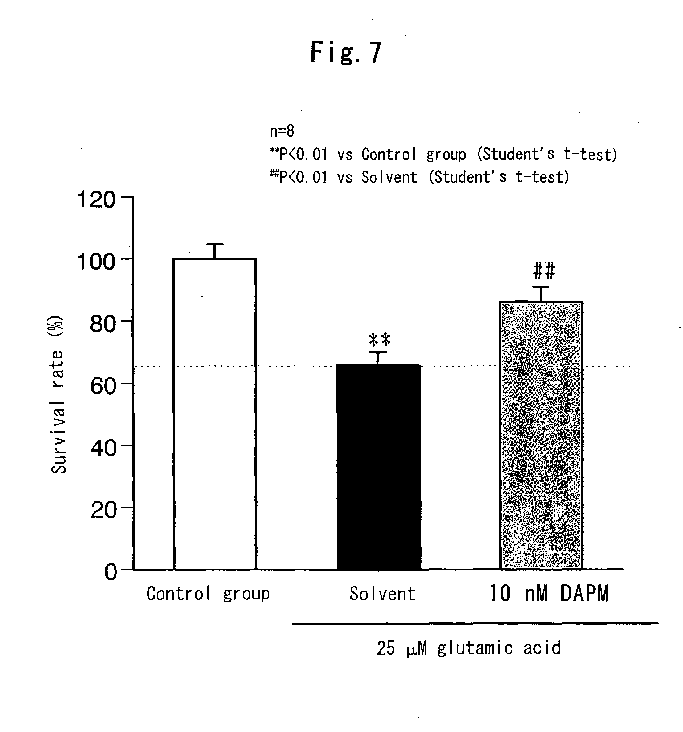

   The same procedure as that described in (1) above was followed except that γXIV (1 nM and 10 nM) was used instead of GL-189, and the survival rate of each group was calculated. The experimental results are shown in the graph of Fig. 4.
(B) Effect on cultured rat neonatal RGC death induced by addition of glutamic acid
   (1) Effect of GL-189
      According to the test method described in Brain Res., 967, 257-66, 2003, RGCs of a rat neonate (at 7 days of age) were isolated by the two-step panning method. As the culture medium, a neuronal culture medium containing a brain derived neurotrophic factor (BDNF, 50 ng/ml), a ciliary neurotrophic factor (CNTF, 50 ng/ml), L-glutamine (2 mM), penicillin/streptomycin (100 U/ml and 100 µg/ml), forskolin (5 µM) and B-27 supplement was used. After the isolation of RGCs, the cells were incubated for 24 hours in a culture medium containing a solvent (0.1% dimethylsulfoxide) or GL-189 (1 µM). Then, glutamic acid was added to give a final concentration of 25 µM in the culture solution and incubation was further carried out for 2 days. The incubation was carried out under the conditions of 37°C and 5% CO₂. As for the control group, incubation was carried out in a culture medium containing a solvent (0.1% dimethylsulfoxide) for the same period of time. After the glutamic acid treatment, identification of live RGCs was carried out using Calcein-AM. More specifically, the cells were incubated for 15 minutes in 1 µg/ml of Calcein-AM solution and washed with a phosphate buffer saline. A cell which is positive for calcein and has a neurite with a length equal to or greater than the diameter of the cell was considered to be a live RGCs, and the number of live RGCs was counted under a fluorescence microscope, and then the survival rate was calculated by taking the case where the number of live RGCs was the same as in the control group as 100%. The experimental results are shown in the graph of Fig. 5. The columns in the graph represent the average value for each group, and the error lines represent the standard error.
   (2) Effect of Z-VLL-CHO
      The same procedure as that described in (1) above was followed except that Z-VLL-CHO (100 nM) was used instead of GL-189, and the survival rate of each group was calculated. The experimental results are shown in the graph of Fig. 6.
   (3) Effect of DAPM
      The same procedure as that described in (1) above was followed except that DAPM (10 nM) was used instead of GL-189, and the survival rate of each group was calculated. The experimental results are shown in the graph of Fig. 7.
   (4) Effect of γXIV

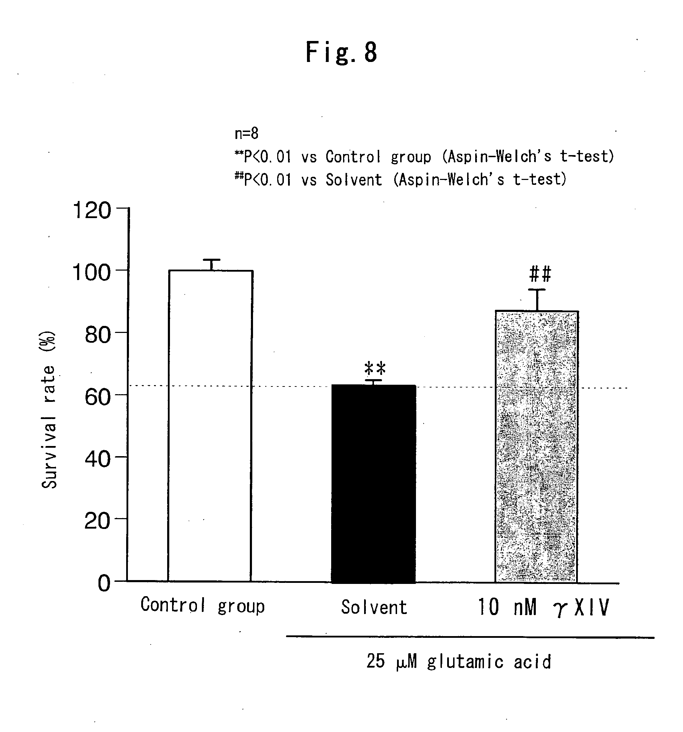

   The same procedure as that described in (1) above was followed except that γXIV (10 nM) was used instead of GL-189, and the survival rate of each group was calculated. The experimental results are shown in the graph of Fig. 8.
(C) Effect of DAPM and Z-VLL-CHO on retinal disorder induced by compression of axon in rat

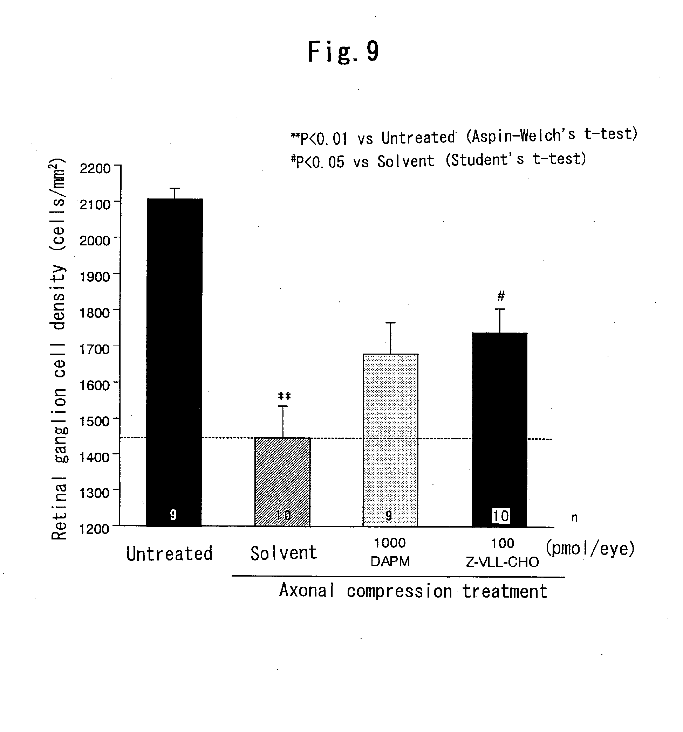

Retrograde labeling of RGCs was carried out by injection of 5% FluoroGold into the midbrain superior colliculus of a rat according to the test method described in J. Ocul. Pharmacol. Ther., 18, 241-9, 2002. At 7 days after the FluoroGold injection, the rat was dilated with the atropine ophthalmic solution, and anesthesia was induced with 3% halothane and maintained with 1% halothane (halothane was vaporized in oxygen at a flow rate of 0.5 L/min and nitrous oxide at a flow rate of 1.5 L/min). The optic nerve was exposed under a stereoscopic microscope. Then, the axon at a distance of 1 to 2 mm from the eyeball was pinched with a clip for 30 seconds at a pressure of 60 g to perform axonal compression treatment. At 5 minutes after the axonal compression treatment, a solvent (0.1% dimethylsulfoxide), DAPM (1000 pmol/eye) or Z-VLL-CHO (100 pmol/eye) was intravitreally administered. At 7 days after the axonal compression treatment, the eyeball was enucleated and fixed in 4% p-formaldehyde solution at 4°C for 1 hour. The cornea, the iris and the lens were removed. The retina was fixed in 4% p-formaldehyde solution at 4°C for an additional 30 minutes, and divided into six pieces in the petal shape to prepare flat-mount specimens. By using a fluorescence microscope and a CCD camera, images whose centers lie on the area at a distance of about 1.5 mm from the optic papilla in each piece obtained by dividing the retina into six and the area at a distance of 0.5 mm from the end of the retina were obtained, respectively (a total of 12 images for each retina). Then, analysis was carried out by using an image analysis software, Optimas, and an average RGC density was calculated. The experimental results are shown in the graph of Fig. 9. The columns in the graph represent the average value for each group, and the error lines represent the standard error.

### [Preparation Examples]

### Preparation Example 1 (Eye drop)

| | | |
|---|---|---|
| Formulation: | GL-189 | 1 mg |
| | Concentrated glycerin | 250 mg |
| | Polysorbate 80 | 200 mg |
| | Sodium dihydrogenphosphate dihydrate | 20 mg |
| | 1 N sodium hydroxide | q.s. |
| | 1 N hydrochloric acid | q.s. |
| | Sterile purified water | q.s. |
| | Total | 10 ml |

To sterile purified water, GL-189 and the above-mentioned components other than GL-189 were added and mixed thoroughly to prepare an eye drop.

### Preparation Example 2 (Tablet)

| | | |
|---|---|---|
| Formulation: | DAPM | 1 mg |
| | Lactose | 66.4 mg |
| | Corn starch | 20 mg |
| | Carboxymethylcellulose calcium | 6 mg |
| | Hydroxypropylcellulose | 6 mg |
| | Magnesium stearate | 0.6 mg |
| | Total | 100 mg |

DAPM, lactose and corn starch were mixed in a mixer, and then carboxymethylcellulose calcium and hydroxypropylcellulose were added to the mixture and granulated. The resulting granules were dried and sized, and then magnesium stearate was added to the sized granules followed by mixing. Then, the mixture was compressed into a tablet with a tableting machine.

### Industrial Applicability

The present invention is directed to a protective agent for retinal neurons comprising a secretase inhibitor as an active ingredient and in particular a therapeutic agent useful for the treatment of a retinal disease typified by retinal vessel occlusion, diabetic retinopathy, ischemic optic neuropathy, macular degeneration, retinitis pigmentosa and Leber's disease or an eye disease accompanied by an optic neuropathy such as glaucoma is provided.

## Claims

1. A protective agent for a retinal neuron comprising a secretase inhibitor as an active ingredient.

2. A therapeutic agent for an eye disease accompanied by an optic neuropathy comprising a secretase inhibitor as an active ingredient.

3. The therapeutic agent according to Claim 2, wherein the secretase inhibitor is a β-secretase inhibitor or a γ-secretase inhibitor.

4. The therapeutic agent according to Claim 2, wherein the eye disease is glaucoma.

5. The therapeutic agent according to Claim 2, wherein the eye disease is a retinal disease.

6. The therapeutic agent according to Claim 5, wherein the retinal disease is retinal vessel occlusion, diabetic retinopathy, ischemic optic neuropathy, macular degeneration, retinitis pigmentosa or Leber's disease.
